# EUROPEAN PATENT APPLICATION

(11) **EP 4 438 097 A1**
(43) Date of publication of application: **02.10.2024**
(21) Application number: 23315072.1
(22) Date of filing: 31.03.2023
(51) Int. Cl.: A61M 25/01, A61M 25/09

(54) **A LOADING UNIT, A LOADING SYSTEM COMPRISING THE LOADING UNIT, AND A METHOD FOR NESTING MEDICAL INSTRUMENT WITHIN EACH OTHER**

(71) Applicant: Caranx Medical, 75013 Paris (FR)
(72) Inventor: CERRUTI, Giulio, 06700 Saint-Laurent-Du-Var (FR); SCHEGG, Pierre, 06000 Nice (FR); SMITS, Jonas Victor Harmen, 3360 Bierbeek (BE)
(74) Representative: Hepp Wenger Ryffel AG

(57) **Abstract**

The invention relates to a loading unit (101) for nesting medical instruments (1, 2) within each other. The loading unit (101) comprises a gripping unit (3, 38) which is convertible between a gripping configuration (32) for radially gripping a first medical instrument (1) and a release configuration (31) for releasing the first medical instrument (1). The loading unit (101) has a translational motion unit (5) for translationally advancing and retracting the first medical instrument (1) in a longitudinal direction (L) of the first medical instrument (1). The loading unit (101) has a centering unit (4) adapted for aligning a longitudinal section of the first medical instrument (1), in particular together with the gripping unit (3, 38) and/or the translational motion unit (5), between the gripping unit (3, 38) and the centering unit (4) along a predefined axis (P). The loading unit (101) has a straightening means (8) for converting the first medical instrument (1), in particular a proximal or distal tip of the first medical instrument (1), from a curved configuration (13) to a straight configuration (14). A control unit (6) of the loading unit (101) is adapted for operating the straightening means (8), the translational motion unit (5), and the gripping unit (3, 38) for advancing a first medical instrument (1) in the straight configuration (14) into a cavity (9) of a second medical instrument (2) or over the second medical instrument (2).

## Description

The invention relates to a loading unit, a loading system comprising the loading unit, and a method for nesting medical instruments within each other using a loading unit according to the independent claims.

Many endovascular interventions, in particular transcatheter aortic valve implantation/replacement (TAVI/TAVR) or coronary angioplasty, require an insertion of a medical instrument into another medical instrument or advancing a medical instrument over/onto another medical instrument.

Medical instruments, such as guidewires, catheters, in particular sheaths and/or dilators, require prior selection and engagement, alignment, and straightening of a medical instrument to allow longitudinal insertion/advancement over another medical instrument to be nested within each other. This may be particularly important throughout a sophisticated endovascular intervention which requires changing a medical instrument, such as differently shaped guidewires, multiple times during the endovascular intervention.

These procedures are usually performed by clinicians who, like the patients, may be exposed to high radiation doses, e.g. from an angiography device, when aligning medical instruments.

It should also be noted that human error, especially in prolonged intravascular interventions, should be minimized through standardization of the workflow and preferably at least partial automation.

US 2012/210569 A1 discloses a guidewire loading tool for a catheter which has a guidewire insertion end and a catheter insertion end connected by a continuous lumen having tapered regions such that a user may insert a guidewire across the lumen into the catheter.

US 2009/118707 discloses a guidewire straightener assembly which has a guidewire tube with a tubular passage which increases in diameter and a guidewire straightener which has a passageway for receiving a guidewire and a finger for frictionally engaging the guidewire.

However, there is generally a lack of a simple and intuitive loading unit for medical instruments which allows for loading, straightening, and exchange of medical instruments which does not rely on the presence of a clinician, and in particular operation by a clinician.

In addition, the prior art lacks an active mechanism which is not reliant on the clinician's skill/attention/learning curve for insertion/advancement of nested medical instruments.

It is the object of the present invention to overcome these and other disadvantages of the prior art, and in particular provide a loading unit, system, and method which allows for nesting medical instrument in a partially, in particular in a completely automated manner. The invention shall further provide a loading unit, system, and method which provides improved handling when exchanging the medical instruments required in an intravascular procedure and preferably facilitating the intervention for the clinician and patient.

These objects are solved by a loading unit, system, and method for nesting medical instruments as defined in the independent claims. Further embodiments result from the dependent claims.

A surgical loading unit for nesting medical instrument within each other according to the invention comprises a gripping unit which is convertible between a gripping configuration for radially gripping a first medical instrument and a release configuration for releasing the medical instrument, in particular in a radial direction. The loading unit has a translational motion unit for translationally advancing and retracting the first medical instrument in a substantially longitudinal direction of the first medical instrument. The loading unit has a centering unit which is adapted for substantially aligning a longitudinal section of the first medical instrument, in particular together with the gripping unit and/or translational motion unit, between the gripping unit and the centering unit along a predefined axis. The loading unit has straightening means for converting the first medical instrument, in particular a proximal or distal tip of the first medical instrument, from a curved configuration to a straight configuration and a control unit which is adapted for operating the straightening means, the translational motion unit, and the gripping unit for advancing a first medical instrument in the straight configuration into a cavity of a second medical instrument or over the second medical instrument.

The medical instrument(s) may comprise or consist of an interventional or diagnostic instrument, in particular an imaging instrument, a drug delivery instrument, a sensory instrument, and/or a medical instrument such as a catheter, a dilator, a sheath, a guidewire, a puncturing element, such as a cannula, and an implantation device.

The alignment/movement of an element of the loading unit, such as the medical instrument(s), along a given axis or direction in the context of this application may be within a certain variation/tolerance, in particular within 1°, 5°, 10°, or 15° angular variation/tolerance with respect to the given axis or direction.

The straightening means may be formed by the centering unit and/or the gripping unit, in particular by the centering unit and/or gripping unit which are controlled in a specific manner via the control unit, in particular by translational motion of the medical instrument via the translational motion unit.

The control unit may be adapted for controlling the translational motion unit for successively advancing and retracting the first medical instrument through the centering unit and/or gripping unit for converting the first medical instrument from the curved configuration to the straight configuration.

This allows the control unit together with the translational motion unit, gripping unit and/or the centering unit to form the straightening means and thus forming a simple design with reduced complexity of the loading unit. This may in particular allow for a stationary centering unit, e.g. such that the centering unit and gripping unit are not translationally movable with respect to each other.

The control unit may be adapted to advance the first medical instrument a predetermined advancement distance in the longitudinal direction across the centering unit. The control unit may further be adapted to retract the medical instrument a predetermined retraction distance, in particular corresponding to arranging the medical instrument within the centering unit, preferably corresponding to a position at which the proximal or distal tip of the first medical instrument is arranged at or within a proximal or distal end of the centering unit. These predetermined distances can allow optimal positioning of the medical instrument if the initial position of the surgical element is known, without the need of a positioning sensor.

Alternatively or additionally, the loading unit may comprise a position sensor for detecting if the proximal or distal tip of the medical instrument is positioned at or within a proximal or distal end of the centering unit connected to the control unit.

The control unit may be adapted for converting the centering unit between a centering configuration for radially engaging and aligning the longitudinal section of the first medical instrument in the centering unit along the predefined axis and an open configuration, in particular by a plurality of iris diaphragms, for radially releasing the first medical instrument.

This allows to advance the first medical instrument across the centering unit in the releasing configuration in which the centering unit is preferably formed and shaped to allow for advancement of the insertion instrument in a plurality of different spatial trajectories. When the insertion instrument was advanced across the centering unit, the centering unit in the centering configuration can precisely engage and center the first medical instrument along the predefined axis.

The gripping unit may be movable orthogonally with respect to the predefined axis such that a gripping spatial position of a medical instrument may be alignable with the predefined axis. The centering unit can comprise a first centering element and a second centering element which are spaced apart from each other along the predefined axis. The first and second centering element may be arrangeable at different radial position with respect to the first medical instrument, preferably on opposite sides of the first medical instrument, and adapted for engaging the first medical instrument in the centering configuration.

The first centering element and the second centering element may be spaced apart from each other in a radial direction with respect to the predefined axis such that an interdistance between the first and second centering element can be adjusted to convert the centering unit between the centering and release configuration.

The centering element may have a concave or wedge-shaped open shape and the second centering element may preferably have a concave or wedge-shaped open shape. The first and second centering element may be at least partially arranged adjacent from each other along a parallel axis to the predefined axis in the centering configuration.

This allows that the concave or wedge-shaped shape defines a receiving section in a two-dimensional plane orthogonal to the predefined axis for receiving the first medical instrument. The concave or wedge-shaped shapes of the centering elements may be arranged towards each other such that the receiving sections can enclose the medical instrument.

By placing the second centering element at least partially adjacent to the first centering element along a parallel axis to the predefined axis, the first medical instrument may be arranged in a predefined spatial position, in particular a predefined spatial position arranged on the predefined axis.

The centering unit may be formed by two halves. A first halve may comprise a plurality of first centering elements and a second halve may comprise a plurality of second centering elements.

The first and second centering elements may form a plurality of teeth that at least partially intermittently engage when the centering unit is converted from the release configuration to the centering configuration.

The teeth allow to provide a centering unit that can contact the medical instruments at several longitudinal sections and center the medical instruments along the predefined axis in a simple and reliable manner.

The centering unit may have a funnel shape with an open end arranged or arrangeable towards the gripping unit. The centering unit may also comprise an additional funnel shape with an open end on the opposite side of the centering unit.

The funnel shape may allow for easier nesting of medical instruments when retracting/advancing medical instruments. In addition, the funnel shape may allow using the centering unit in combination differently shaped medical instruments, e.g. a preshaped surgical guidewire with a curved proximal or distal tip or a medical instrument with a hub with a larger cross-sectional dimension which may be formed correspondingly to the funnel shape.

The funnel shape may also be correspondingly formed to receive a protrusion of another centering unit which is configured to surround the first medical instrument at least partially.

The translational motion unit may be adapted for driving the gripping unit, in particular a pair of rotating rollers of the gripping unit, for advancing and retracting the first medical instrument in the longitudinal direction. Alternatively or additionally, the translational motion unit may be adapted for moving the gripping unit with respect to the centering unit and/or the centering unit with respect to the gripping unit.

The translational motion unit may comprise a rotational actuator for rotating the pair of rotating rollers of the gripping unit. The translational motion unit may comprise a translational actuator for translationally moving e.g. a lever on which the gripping unit, the centering unit and/or in particular the straightening means is arranged.

The loading unit may comprise a first translational motion unit for advancing and retracting the first medical instrument in the longitudinal direction and a second translational motion unit for moving the gripping unit with respect to the centering unit and/or the centering unit with respect to the gripping unit.

The loading unit may have an additional translational motion unit adapted for advancing and retracting a core element into a longitudinal lumen of the first medical instrument. Alternatively, the additional translational motion unit may be adapted for advancing and retracting a sheath which at least partially encloses the first medical instrument circumferentially over the first medical instrument. The advancement of the core/sheath by the second translational motion unit, in particular along the medical instrument, is adapted for converting the first medical instrument from the curved configuration to the straight configuration.

This allows for simple alignment and conversion of the first medical instrument to the straight configuration, and in particular for at least partially automated straightening as the core/sheath can preferably be easily aligned along the predefined axis when being inserted into/advanced over the first medical instrument.

The control unit may be adapted for operating the straightening means which interacts with a characteristic of the first medical instrument, in particular a magnetic characteristic, a temperature characteristic, an electric characteristic, or an electro-chemical characteristic, to transition the first medical instrument from the curved configuration to the straight configuration.

The straightening means may be adapted to change the temperature or electric characteristic, e.g. via heating means or electrodes attached adjacent the medical instrument, such that a first medical instrument which comprises a shape memory alloy, may be convertible above a transition temperature to be deformed to the straight configuration.

The straightening means adapted for changing an electro-chemical characteristic may be adapted to apply a voltage or an electric field to convert a first medical instrument which comprises an ionic polymer-metal composites between the curved and straight configuration by ion migration/redistribution.

A system comprising the loading unit may include one of the previously described first medical instruments having a characteristic for converting between the curved and straight configuration.

The gripping unit may comprise or consist of a pinch gripper, in particular a pinch gripper formed by at least two cylindrical rollers, preferably having a centering surface, in particular a partially helical centering surface or two conical centering surfaces forming a V-shape, for engaging the first medical instrument in the gripping configuration.

The cylindrical rollers may be adapted to translationally move the first medical instruments by engaging the first medical instrument from either side and rotation of the cylindrical rollers in opposite directions around a symmetry axis of the cylindrical rollers.

The cylindrical roller may have two helical centering surfaces which have opposite left-/right-handed winding directions. The left-handed helical centering of the first cylindrical roller may be placed adjacent the right-handed helical centering surface of the second cylindrical roller and vice versa.

Alternatively or additionally the cylindrical rollers may have a V-shaped centering surface. In particular, the cylindrical rollers may have an hourglass shape which may be rotationally symmetric around its longitudinal axis.

This allows the medical instrument to be moved to a predefined center position when the cylindrical rollers are rotated in one direction. The center position of the cylindrical roller may be recessed and/or extend only in a circumferential direction of the cylindrical roller to retain the first medical instrument in the center position during further rotation of the cylindrical roller in both directions.

The gripping unit may comprise or consist of a self-centering gripper for engaging the first medical instrument with at least three, in particular exactly three, contacting areas of the self-centering gripper in the gripping configuration.

A self-centering gripper with three contact areas allows the contact areas to be arranged for engagement with the first medical instrument in such a way that it can be securely fastened in a predefined centering position while at the same time allowing the first medical instrument to be reliably detachable/releasable, in particular in a radial direction. The predefined centering position preferably is arranged or arrangeable along the predefined axis.

The gripping unit may comprise or consist of a hook gripper which has an open shape in the release configuration. The open shape, in particular a monolithic open shape, is configured for radially receiving the first medical instrument in a predetermined position. The at least partially closed shape in the gripping configuration of the hook gripper is shaped for preventing radially releasing the first medical instrument.

The gripping unit may comprise or consist of a gripper for radially engaging the first medical instrument at two different longitudinal locations of the first medical instrument in the gripping configuration to center the first medical instrument, in particular along the predefined axis.

These gripping units allow for secure engagement of the first medical instrument and automatic centering of the first medical instrument once it was engaged by the gripping unit in the gripping configuration.

The loading unit may have a gripping unit positioner which is adapted to position the gripping unit in a plane orthogonal to the predefined axis in one degree of freedom, in particular in two degrees of freedom, such that different medical instruments may be engaged/exchanged and/or positioned along the predefined axis.

The self-centering gripper may include a mechanical coupling for converting the self-centering gripper by a rotational or translational motion between the gripping configuration for gripping the first medical instrument in which the first medical instrument is contacted by the at least three contact areas and the release configuration for disengaging the first medical instrument.

This mechanical coupling allows a simplified control of the self-centering gripper for centering the first medical instrument.

The loading unit, in particular the self-centering gripper/translational motion unit, may comprise an actuator, in particular a rotational or translational actuator for converting the self-centering gripper between the release and gripping configuration.

In the gripping configuration, the at least three contact areas may be arranged such that an angle between the contact area gripping the first medical instrument extends over a range greater than 180° in a circumferential direction of the first medical instrument in total. This allows for a secure grip of the first medical instrument and prevents an accidental radial release of the first medical instrument.

Angles between the contact areas may be greater than 60°, in particular greater than 90°, preferably 120°.

The contact areas may be circumferentially equidistantly spaced apart from each other such that they are arranged at essentially the same angle with respect to each other. For example, two contact areas may be arranged at 180° to each other, three contact areas at an angle of 120° with respect to each other, four contact areas at an angle of 90° with respect to each other, five contact areas at an angle of 72° with respect to each other, or six contact areas at an angle of 60° with respect to each other.

The contact areas of the gripping unit may be arranged at a distal tip an arm or a plurality of arms of the gripping unit. The arm or plurality of arms may be tiltable around a joint of the gripping unit and/or translationally advanceable/retractable.

The gripping unit may comprise at least one elastic element, in particular a spring element, for applying a spring force in a radial direction on the first medical instrument. The elastic element may be connected to the at least one arm, in particular the plurality of arms, of the gripping unit.

The elastic elements allow for secure engagement of the first medical instrument by enabling a secure grip on the first medical instrument with a predetermined frictional force. In addition, an elastic element may allow to securely grip a plurality of medical instrument which have different cross-sectional dimensions.

The control unit and/or the translational motion unit may be adapted for translationally moving and/or tilting one arm, in particular at least two arms, preferably three arms, when converting the gripping unit between the gripping configuration and release configuration.

A loading system comprising a loading unit as previously described may comprise a second gripping unit which is convertible between a gripping configuration for radially gripping a second medical instrument and a release configuration for releasing the second medical instrument. The loading system further has a second translational motion unit for translationally advancing and retracting the second medical instrument in a longitudinal direction of the second medical instrument. The loading system has a second centering unit adapted for aligning a longitudinal section of the second medical instrument, in particular together with the gripping unit, between the second gripping unit and the second centering unit along the predefined axis. The loading system has a second straightening means for converting the second medical instrument, in particular a proximal tip of the second medical instrument from a curved configuration to a straight configuration. The control unit is adapted for operating the second straightening means, the second translational motion unit, and the second gripping unit for nesting the first medical instrument and the second medical instrument within each other. The centering units of the loading system are arranged or arrangeable between the gripping units.

Within the present application, the proximal and distal directions are defined as the proximal direction toward an operator/clinician of the loading unit and the distal direction away from the operator/clinician and towards a patient.

This allows to arrange the first and second medical instrument along the predefined axis in the loading procedure when nesting the medical instruments in a partially, in particular fully, automated manner without requiring the expertise/capacities of a clinician.

The second straightening means, the second translational motion unit, the second centering unit and the second gripping unit may have the same previously described features as the first straightening means, the first translational motion unit, the first gripping unit and the first centering unit.

The centering units, in particular with the gripping units, of the loading system may be movable along the predefined axis with respect to each other, in particular by a translational motion unit. This allows that the straightened first medical instrument arrangeable within the first centering unit is insertable into the straightened second medical instrument arrangeable within the second centering unit by arranging the centering units adjacent each other.

The movement of the centering units may be controlled via the control unit and preferably carried out by the first or second translational motion unit and/or an additional translational motion unit.

One of the centering units, in particular the first centering unit, may comprise a longitudinal protrusion which is arrangeable to at least partially circumferentially enclose the medical instrument and be at least partially insertable into the other of the centering units.

This allows a more secure connection between the centering units to allow for optimized alignment and nesting of the medical instrument within each other.

Another aspect of the invention relates to a method for nesting medical instruments within each other using a loading unit, in particular a previously described loading unit. The loading unit has a gripping unit which is convertible between a gripping configuration for radially gripping the first medical instrument and a release configuration for releasing the first medical instrument. The loading unit has a translational motion unit for translationally advancing and retracting the first medical instrument in a longitudinal direction of the first medical instrument. The loading unit has a centering unit which is adapted for aligning a longitudinal section of the first medical instrument, in particular together with the gripping unit, between the gripping unit and the centering unit along a predefined axis.

The method comprises converting the gripping unit from the release configuration to the gripping configuration to radially grip the first medical instrument. The method comprises translationally advancing the first medical instrument via the translational motion unit in the longitudinal direction of the first medical instrument across the centering unit and optionally converting the centering unit from an open configuration to a centering configuration. The method comprises translationally retracting the first medical instrument via the translational motion unit in the longitudinal direction of the first medical instrument, such that the first medical instrument, in particular a proximal or distal tip of the first medical instrument, is straightened by the centering unit. The method comprises advancing the first medical instrument into a second medical instrument.

The method may comprise converting a second gripping unit from a release configuration to a gripping configuration to radially grip the second medical instrument. The method further may comprise translationally retracting the second medical instrument via a second translational motion unit in the longitudinal direction of the second medical instrument across a second centering unit. The method optionally comprises converting the second centering unit from the open configuration to the centering configuration. The method may comprise translationally advancing the second medical instrument via the second translational motion unit in the longitudinal direction, such that the second medical instrument, in particular a proximal tip of the second medical instrument, is straightened by the second centering unit. The method optionally comprises moving the first and/or second centering unit, in particular together with the respective first and/or second gripping unit, such that the centering units are arranged adjacent each other before advancing the first medical instrument into or onto the second medical instrument.
- Figure 1:: A schematic representation of loading medical instruments by nesting a first medical instrument within a second medical instrument known in the prior art,
- Figure 2:: A perspective view of an embodiment of the loading unit with a gripping unit, translational motion unit and centering unit,
- Figs. 3A-3B:: Perspective views of a first embodiment and a second embodiment of a straightening means of the loading unit,
- Figs. 4A-4B:: Perspective views of a first and second embodiment of a half centering unit with a plurality of centering elements,
- Figs. 5A-5B:: A top view of an embodiment of the centering unit in the centering and open configuration,
- Figure 5C:: A perspective view of an embodiment of the centering unit in the open configuration,
- Figs. 6A-6B:: A perspective view of an embodiment of a gripping unit formed by a self-centering gripper in a release configuration and gripping configuration respectively enclosing a cross-sectional section of a medical instrument,
- Figs. 7A-7B:: A perspective view of an embodiment of a gripping unit formed by a self-centering gripper in a release and gripping configuration,
- Figure 8A:: A perspective view of an embodiment of half a gripping unit having a cylindrical roller which has two helical centering surfaces,
- Figure 8B:: A perspective view of an embodiment of a gripping unit having two cylindrical rollers which have two helical centering surfaces respectively,
- Figures 8C-8G:: Six different perspective views of an embodiment of half a gripping unit having a cylindrical roller with two conical centering surfaces forming a V-shape,
- Figure 8H:: A cross-sectional view of the cylindrical roller of Figs. 8C-8G,
- Figure 9:: A perspective view of an embodiment of a loading system comprising a loading unit, a second centering unit, and a second gripping unit, a second translational motion unit, and a control unit,
- Figs. 10A-10H:: Perspective view of a method using a loading unit for centering and straightening of a first medical instrument for advancement and insertion into a second medical instrument,
- Figs. 11A-11H:: Perspective view of a method using a loading system comprising a loading unit for centering and straightening a first medical instrument shown in Fig. 9 by advancement and insertion into a second medical instrument.

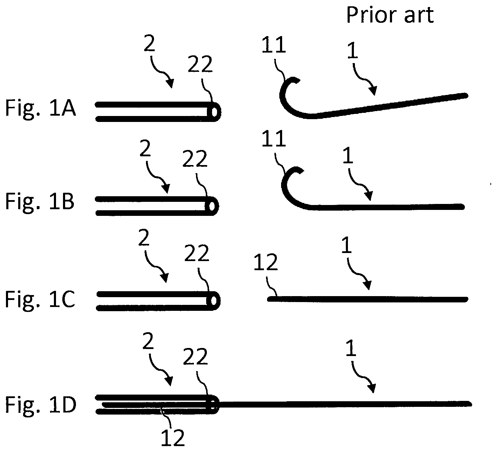

Figure 1 shows a schematic representation of a cross-sectional view of loading medical instruments 1, 2 by nesting a first medical instrument 1 within a second medical instrument 2 known in the prior art. To advance the first medical instrument 1 into a proximal tip 22 of the second medical instrument 2, the first medical instrument 1 is (i) aligned along a common axis with the second medical instrument 2 and (ii) a curved distal tip 11 of the first medical instrument 1 is straightened either manually or by using a passive loading tool as described e.g. in US 2012/210569 A1. However, this requires the presence and takes up resources of a clinician which may lengthen the surgical procedure and may e.g. increase a radiation dose exposure of a patient and the clinician during angiography when changing medical instrument prior to or throughout surgical procedures.

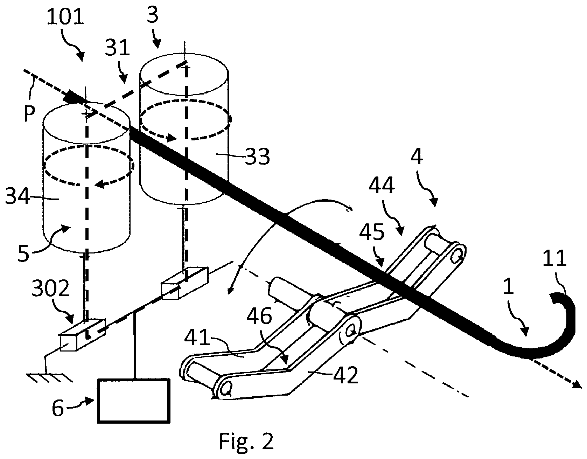

Figure 2 shows a perspective view of an embodiment of the loading unit 101 which has a gripping unit 3, a translational motion unit 5, and a centering unit 4. The gripping unit 3 is formed by a pinch gripper comprising two cylindrical rollers 33, 34 which is convertible between a release configuration 31 and a gripping configuration for radially engaging the first medical instrument 1.

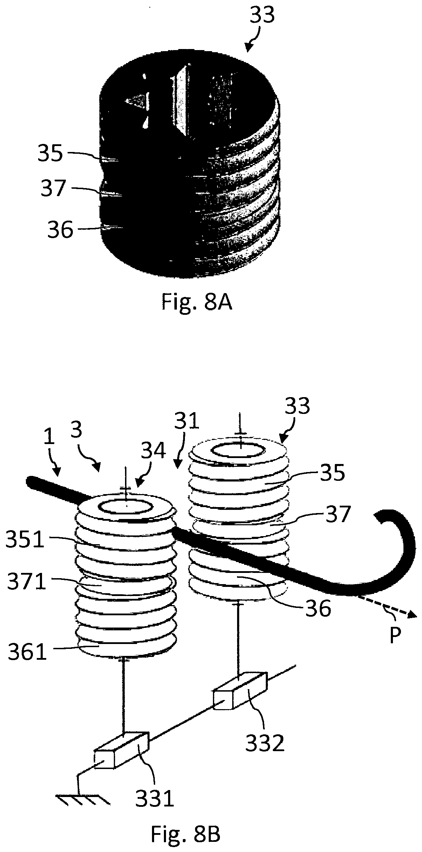

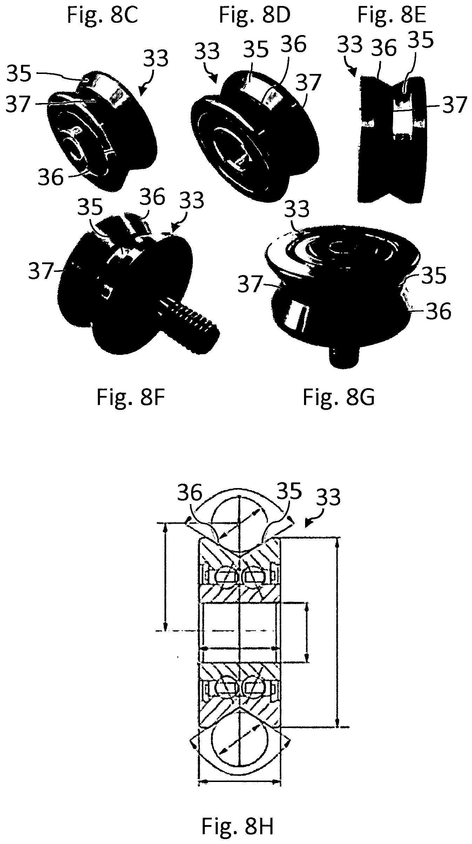

The gripping unit 3 is adjusted for radially engaging the first medical instrument 1 by translational actuators 302 which are included in the translational motion unit 5 moving the first cylindrical roller 33 and second cylindrical roller 34 toward each other. The gripping unit 3 is further connected to two rotational actuators included in the translational motion unit 5 for rotating the first and second cylindrical rollers 33, 34 around their symmetry axis as indicated by the dashed arrow in opposing directions for advancing or retracting the first medical instrument 1. Alternatively, the translational actuators and rotational actuators may be arranged separate from each other, preferably such that the translational actuators are adapted for translationally moving the rotational actuators together with the gripping unit 3. The figure 2 shows that the rotational actuators of the translational motion unit 5 are arranged within the cylindrical rollers 33, 34 for rotating the cylindrical rollers and thus translationally moving the medical instrument 1 in a longitudinal direction. The rotational actuators are movable by converting the gripping unit 3 between a release and a gripping configuration via the translational actuators 302 when engaging/releasing the medical instrument 1. The rotational actuators are preferably arranged within the cylindrical rollers 33, 34. The cylindrical rollers 33, 34 can preferably comprise a self-centering surface 35, 36 (see Figs. 8A and 8H).

The centering unit 4 in Fig. 2 is in the open configuration 44 and is convertible to a centering configuration for radially engaging the first medical instrument 1 as indicated by the bent arrow. The centering unit 4 has a first and a second centering element 41, 42 which are spaced apart from each other with respect to a predefined axis P. The centering elements 41, 42 both have a wedge-shaped open shape 45, 46 with the open side arrangeable around the medical instrument 1. The wedge-shaped open shapes 45, 46 are adapted for centering the first medical instrument along the predefined axis P. In the open configuration 44 of the centering unit 4, the first medical instrument 1 can be advanced across the centering unit 4 via the cylindrical rollers 33, 34 being rotated by the translational motion unit 5 in a plurality of different trajectories. After advancement of the first medical instrument 1, the centering unit 4 can be converted by an automated actuator to the centering configuration for centering the first medical instrument 1.

A control unit 6 is adapted for fully automated control of the gripping unit 3, the translational motion unit 5, and the centering unit 4. The control unit 6 is further adapted to carry out advancement of the first medical instrument 1 across the centering unit 4 and subsequent retraction of the medical instrument 1 through centering unit 4 in the centering configuration for straightening a distal tip 11 of the first medical instrument 1. An advancement and retraction distance are determined by a predetermined advancement/retraction distance based on a known spatial relationship between the centering unit and the medical instrument when being engaged by the gripping unit. Alternatively, a position sensor for detecting a distal tip of the medical instrument 1 may be used for determining the advancement and retraction distance.

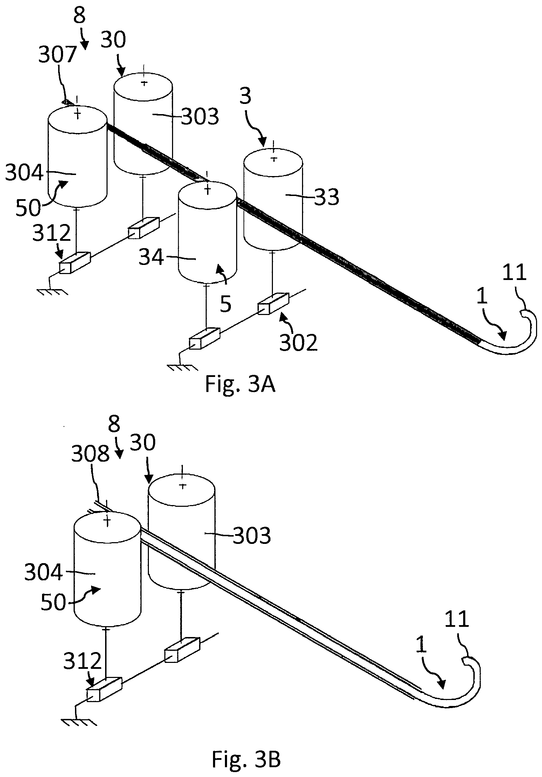

Figures 3A and 3B show perspective views of a first and second embodiment of a straightening means 8 of the loading unit which is formed by a second translational motion unit 50 and a second gripping unit 30 and a straight inner core 307 or straight sheath 308 respectively.

The operation of the first gripping unit 3 with two cylindrical rollers 33, 34, the translational actuators 302, and the translational motion unit 5 in Fig. 3A (not explicitly shown in Fig. 3B) are the same as previously described in Fig. 2.

The second translational motion unit 50 has rotational actuators for rotating the cylindrical rollers 303, 304 of the second gripping unit 30. This allows that the core 307 or sheath 308 in Figs. 3A and 3B can be advanced and retracted with respect to the first medical instrument 1 once being engaged by the gripping unit 30 if the second gripping unit 30 is converted from a release configuration to a gripping configuration. The translational motion units 50 and the gripping unit 30, as well as a translational actuator 312 of the gripping unit 30, are formed as previously described in Fig. 2.

The core 307 and sheath 308 have a straight shape and greater stiffness than the first medical instrument 1 such that the core 307 (Fig. 3A) or the sheath 308 (Fig. 3B) can be advanced into or over the first medical instrument 1 for straightening the first medical instrument 1. The core 307/sheath 308 is advanceable up to a distal tip 11 of the first medical instrument 1 such that the first medical instrument is straightened.

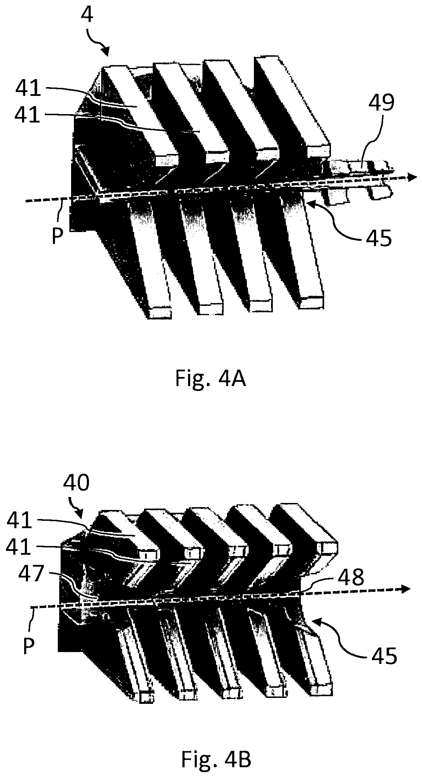

The figures 4A and 4B show perspective views of a first and a second embodiment of one half of a centering unit 4, 40 of the loading unit. The halve of the centering unit 4 has a plurality of centering elements 41 which are arranged spaced apart along a predefined axis P in the centering configuration. The plurality of centering elements 41 form wedge-shaped open shapes 45 for circumferentially engaging the first medical instrument at a plurality of locations along the predefined axis P and centering the first medical instrument along the predefined axis P.

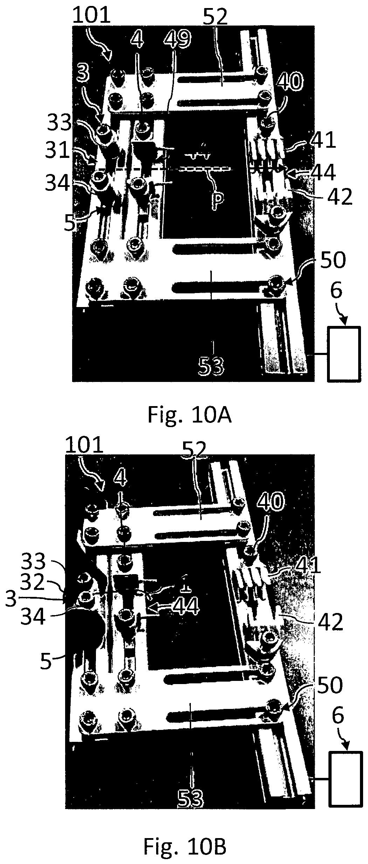

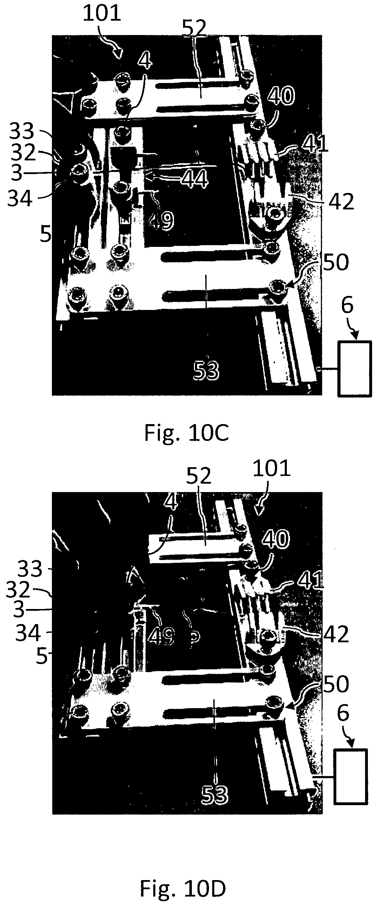

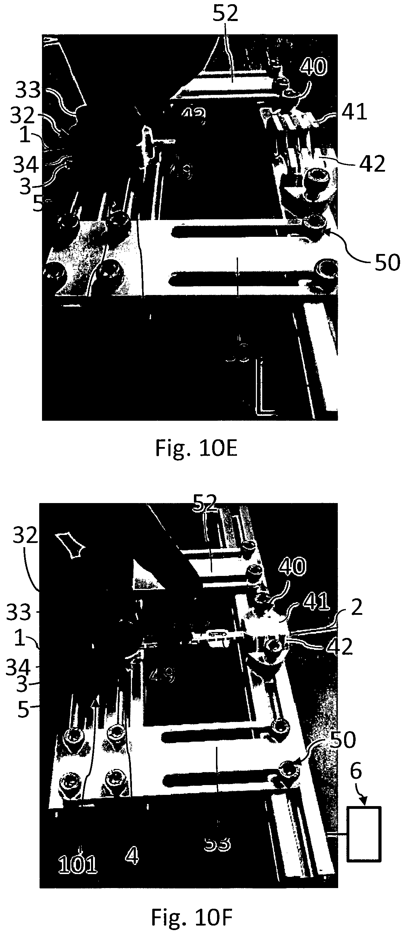

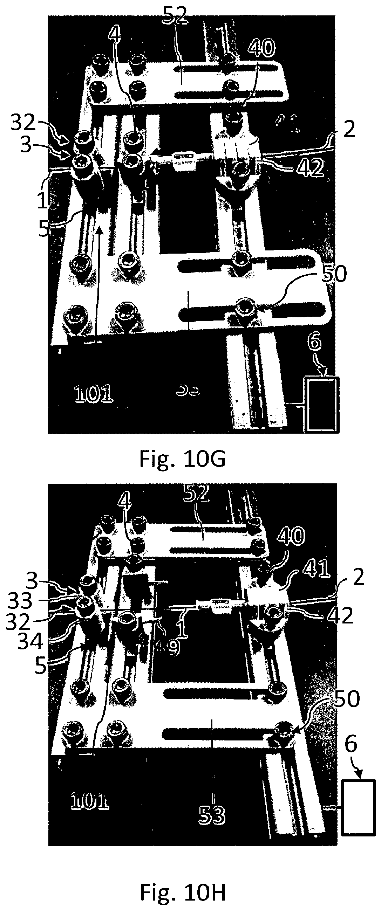

The centering elements 41 in Figs. 4A and 4B are spaced apart by gaps for receiving second centering elements 42 of a second halve of the centering unit 4, 40 (see Fig. 10). Thereby the centering elements 41, 42 form a plurality of teeth which are adapted to intermittently engage each other such that they are partially overlapping along the predefined axis P. This allows for a secure positioning of the first medical instrument within the centering unit 4, 40 along the predefined axis P once the centering unit 4, 40 is converted to the centering configuration.

The halve of the centering unit 4 in Fig. 4A has a distal conical protrusion 49 which is insertable into a funnel shape 47, 48 of the centering unit 40 shown in figure 4B. This allows for secure alignment/attachment of the centering units 4, 40 with respect to each other shown in Figs. 4A and 4B when inserting the first medical instrument, e.g. a guidewire, into a second medical instrument, e.g. a catheter (see Figs. 10A - 11H).

The proximal funnel shape 47 of the centering unit 4 shown in figure 4B can also function as a stop for a cross-sectional widening of a second medical instrument such that the second medical instrument can be retracted proximally across the centering unit 4 in Fig. 4B and subsequently advanced up to the widening of the second medical instrument. Such a widening may allow for a simplified positioning when the widening causes an increased resistance for further retraction. The two funnel shapes 47, 48 on either side may have different cross-sectional dimensions corresponding to different medical instruments. Alternatively, only a proximal or distal funnel shape 47, 48 may be arranged on one side of the centering unit 4. This allows, for example, the second centering unit 40 to be mounted inverted along a predefined axis if another second medical instrument with a different cross-sectional dimension is to be used. In addition, the at least one funnel shape 47, 48 allows for accommodating a protrusion/tip, in particular a conical protrusion/tip, of a medical instrument, in particular a TAVI delivery system, a centering unit 4, or a gripper unit 3.

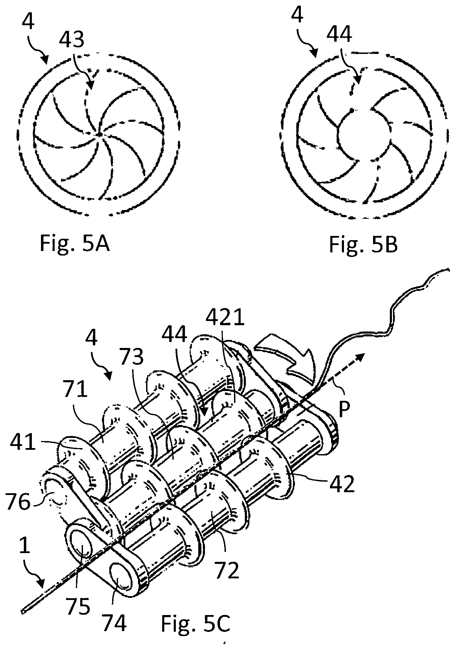

The figures 5A and 5B show a top view of an embodiment of a centering unit 4 formed by a plurality of iris diaphragms.-The plurality of iris diaphragms is arranged along the predefined axis for centering the medical instrument (not shown in Figs. 5A and 5B). The iris diaphragm shown in figure 5A shows a centering configuration 43 and the iris diaphragm in figure 5B shows an open configuration 44 which are convertible between each other by a control unit. The center position of the iris diaphragms is arranged or can be arranged on a predefined axis for centering the medical instrument.

The figure 5C shows a perspective view of an alternative embodiment of a centering unit 4 formed analogous to an industrial wire straightener in an open configuration 44. The centering unit 4 is formed by three parallel struts 71, 72, 73 having three pluralities of centering elements 41, 42, 421 respectively formed by annular protrusions with equal radial dimensions which are spaced apart along a predefined axis P. The centering elements 41, 42, 421 are adapted to engage a medical instrument 1 in a centering configuration of the centering unit 4 to align the medical instrument 1 along the predefined axis P. The struts 71, 72, 73 are connected via hinges 74, 75, 76 which are arranged at a proximal and distal end of the centering unit 4 such that the struts 71, 72, 73 are actuatable by a rotational actuator controlled by a control unit. This allows for converting the centering unit 4 between the open configuration 44 and centering configuration.

The three centering elements 41, 42, 421 on the struts 71, 72, 73 are equidistantly arranged on the respective strut 71, 72, 73. The plurality of centering elements 41, 42, 421 are arranged to be offset along the predefined axis P with respect to the other centering elements 41, 42, 421 of another strut 71, 72, 73 such that the centering elements 41, 42, 421 can intermittently engage each other in the centering configuration. By moving the medical instrument 1 relative to the centering unit 4 enclosing the medical instrument 1 in the centering configuration the medical instrument 1 is straightened and aligned along the predefined axis P.

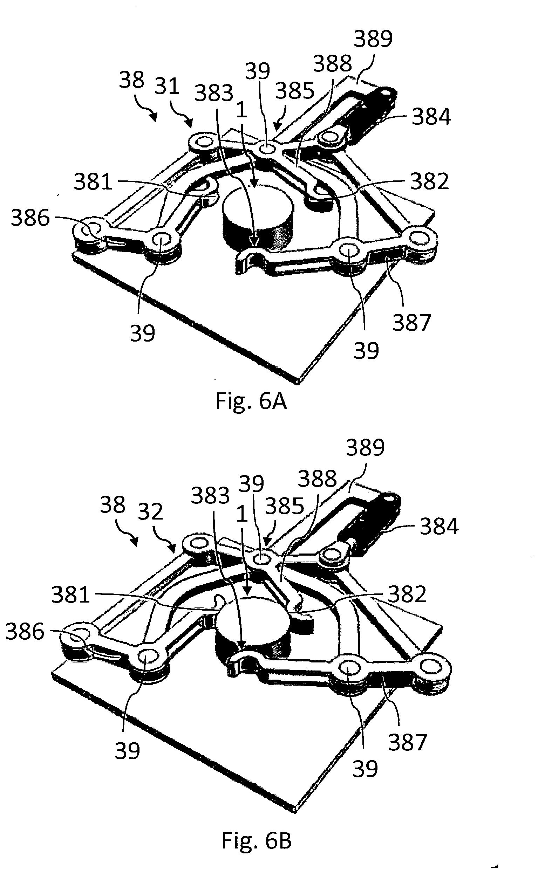

The figures 6A and 6B show a perspective view of an embodiment of a gripping unit formed by a self-centering gripper 38 in a release configuration 31 and a gripping configuration 32 respectively enclosing a longitudinal subsection of a medical instrument 1. The self-centering gripper 38 has three lever arms 386, 387, 388 which are tiltable by actuating a translational actuator 384 for converting the self-centering gripper 38 between the release configuration 31 and the gripping configuration 32. The self-centering gripper 38 has a mechanical coupling 385 formed by a plurality of joints 39 to allow for a tilting movement of the three lever arms 386, 387, 388 by only the one translational actuator 384. The joints 39 are functionally connected to the lever arms 386, 387, 388 and a fixed frame 389 which is not affected by the translational actuator 384. These joints 39 allow the lever arms 386, 387, 388 to be pivoted relative to the fixed frame 389 for redirecting the translational actuation by the translational actuator 384. The mechanical coupling 385 is adapted to convert the translational actuation by the translational actuator 384 such that 3 contacting areas 381, 382, 383 arranged on the ends of the lever arms 386, 387, 388 can engage the first medical instrument 1 in the gripping configuration 32. The contacting areas 381, 382, 383 are arranged to engage the first medical instrument 1 in a circumferential direction of the first medical instrument 1 at 120° with respect to each other in the gripping configuration 32 in Fig. 6B. In the release configuration 31 shown in Fig. 6A, an opening sufficient for radial release of the medical instrument 1 is formed by the self-centering gripper 38 (see Fig. 6A). The translational actuator 384 is operated by a control unit in a fully automated manner.

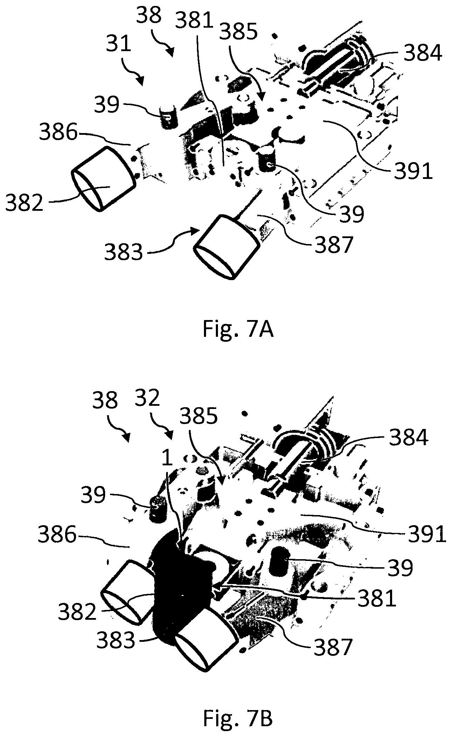

Figures 7A and 7B show a perspective view of an embodiment of a gripping unit formed by a self-centering gripper 38 in a release configuration 31 and gripping configuration 32.

The self-centering gripper 38 has a translational actuator 384 which is adapted to drive a triangular coupling element 391 to tilt two lever arms 386, 387 around joints 39 for converting the self-centering gripper 38 between a release configuration 31 and a gripping configuration 32. In addition, translation of the triangular coupling element 391 advances a first contacting area 381 towards the medical instrument 1 while at the same time radially engaging the medical instrument 1 by a second contacting area 382 and third contacting area 383 by tilting the lever arms 386, 387.

The figure 7A shows that the self-centering gripper 38 in the release configuration 31 allows for radially engaging/releasing the medical instrument 1 by having outwardly tilted lever arms 386, 387. The figure 7B shows the contacting areas 381, 382, 383 of the self-centering gripper 38 engage a medical instrument 1 in the gripping configuration 32 in a circumferential direction of the first medical instrument 1 at 120° with respect to each other.

The contacting areas 381, 382, 383 are formed by rollers, preferably cylindrical rollers, which are adapted to circumferentially engage the medical instrument 1 with a lateral surface of the rollers. The rollers are further be adapted for translationally moving the medical instrument 1 in the gripping configuration 32 of the self-centering gripper 38 by rotating around an axis orthogonally with respect to the predetermined axis.

The self-centering gripping unit of Figs. 6A - 7B is arranged on a gripping unit positioner (not shown in the figures) such that the gripping unit is movable in a plane orthogonal to a predefined axis for changing/engaging the medical instruments. This allows for engaging and switching medical instruments in a fully automated manner.

The figure 8A shows a perspective view of an embodiment of one halve of a gripping unit 3 having a cylindrical roller 33 which has two helical centering surfaces 35, 36. The helical centering surfaces 35, 36 have grooves for receiving a medical instrument. The helical centering surfaces 35, 36 are arranged with opposing left-/right-handed orientation, such that rotation a rotation of the cylindrical roller 33 in one direction is adapted to move a medical instrument closer to a center position 37 which is recessed and extends only in a circumferential direction of the cylindrical roller 33 such that the medical instrument can be retained in the center position 37 irrespective of the direction of rotation of the cylindrical roller 33.

The figure 8B shows a perspective side view of an embodiment of a gripping unit 3 having a first and second cylindrical roller 33, 34 with two helical centering surfaces 35, 36; 351, 361 respectively in a release configuration 31. The first and second cylindrical rollers 33, 34 are arranged on a first and second rotational actuator 331, 332 such that the cylindrical rollers 33, 34 are rotatable around their symmetry axis. This allows for advancing and retraction of the medical instrument 1 in the gripping configuration when the cylindrical rollers 33, 34 rotate in opposing directions. The gripping unit 3 is convertible between the release configuration 31 and the gripping configuration by a translational actuator for moving the cylindrical rollers 33, 34 in a radial direction closer together or further apart (not shown in Fig. 8B).

Fig. 8B shows that the helical centering surfaces 35, 36; 351, 361 of the cylindrical rollers 33, 34 are arranged such the first helical centering surface 35 of the first cylindrical roller 33 which has a left-handed orientation is arrangeable neighboring the first helical centering surface 351 of the second cylindrical roller 34 which has a right-handed orientation. Accordingly, the second helical centering surface 36 of the first cylindrical roller 34 which has a right-handed orientation is arranged neighboring the second helical centering surface 361 which has a left-handed orientation. This allows that opposing rotation of the cylindrical rollers 33, 34 for advancing the medical instrument 1 in a distal direction of the medical instrument 1 in the gripping configuration moves the medical instrument 1 to the centering positions 37, 371of the cylindrical rollers 33, 34. The centering positions 37, 371 are positioned along the predefined axis P such that the medical instrument 1 can be aligned with the predefined axis P. The actuators for controlling the conversion of the gripping unit 3 and advancement/retraction of the medical instrument 1 are controlled by a control unit 6 (see Fig. 2) in an automated manner.

The figures 8C-8G show six different perspective views of an embodiment of half a gripping unit 3 having a cylindrical roller 33 with two conical centering surfaces 35, 36 forming a V-shape. The V-shape of the two lateral centering surfaces 35, 36 of the cylindrical roller 33 allows to center a medical instrument within a recessed center position 37 when engaging the medical instrument from opposing sides with two cylindrical rollers 33, 34 (see e.g. Fig. 8A).

The figure 8H shows a cross-sectional view of the cylindrical roller 33 of Figs. 8C-8G without a recessed center position.

The Figs. 8A-8H show that the cylindrical roller 33 may have a centric passage or for receiving a spindle to be coupled to a rotational actuator. In addition, the cylindrical roller 33 may comprise a ball bearing.

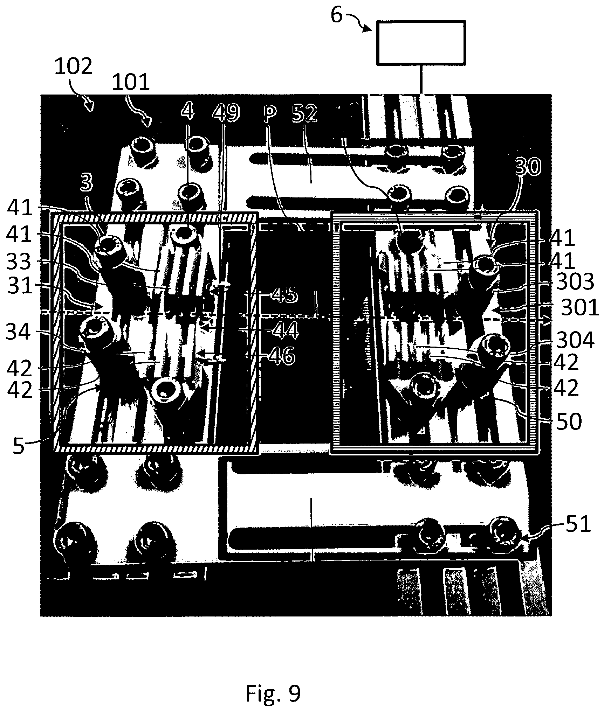

The figure 9 shows a perspective view of an embodiment of a loading system 102 comprising a loading unit 101 which has a first gripping unit 3, a first centering unit 4, and a first translational motion unit 5. The loading system 102 in Fig. 9 further has a second gripping unit 30, a second centering unit 40, a second translational motion unit 50, and a control unit 6.

The gripping units 3, 30 in Fig. 9 are in the release configuration 31, 301 and have two cylindrical rollers 33, 34; 303, 304 respectively which are adapted to radially engage medical instruments. The gripping units 3, 30 may alternatively comprise a plurality of pairs of cylindrical rollers 33, 34; 303, 304 which are longitudinally spaced apart from each other and are adapted for radially engaging medical instruments. Similarly, as previously described in Fig. 2, the cylindrical rollers 33, 34; 303, 304 are connected to translational motion units 5, 50 respectively such that the medical instruments can be advanced and retracted by rotational actuation of the cylindrical rollers 33, 34; 303, 304 in opposing directions along a longitudinal direction of the medical instruments. The cylindrical rollers 33, 34; 303, 304 are also movable perpendicular to a predefined axis P toward each other or apart from each other such that the gripping units 3, 30 can be converted between the release configuration 31, 301 and gripping configuration.

The first and second centering units 4, 40 are arranged between the first and second gripping units 3, 30 such that two medical instruments 1, 2 can be nested within each other when the centering units 4, 40 are neighboring each other (see e.g. Figs. 10A - 11H). Both centering units 4, 40 in Fig. 9 are in the open configuration 44 and have a plurality of first and second centering elements 41, 42 which are arranged spaced apart from each other along the predefined axis P. In the centering configuration 44, the open wedge-shape 45, 46 formed by the centering elements 41, 42 of the centering units 4, 40 intermittently interlock such that a medical instrument can be centered along the predefined axis. In addition, the medical instrument can be straightened by being advanced/retracted by the cylindrical rollers 33, 34; 303, 304 driven by the translational motion unit 5, 50 by passing through the centering units 4, 40.

The first centering unit 4 is formed by two intermittently engageable halves described in Fig. 4A which has a conical protrusion 49. The second centering unit 40 is formed by two intermittently engageable halves described in Fig. 4B with two funnel shapes 47, 48 (not shown in Fig. 9).

The first centering unit 4 and the first gripping unit 3 (characterized by a square with a diagonal hatching) are movable by a third translational motion unit 51 with respect to the second centering unit 40 and the second gripping unit 30 (characterized by a square with a horizontal hatching). The third translational motion unit 51 is adapted to move the first centering and gripping unit 4, 3 by moving parallel struts 52, 53 to which the centering and gripping unit 4, 3 are attached to parallel to the predefined axis P. This allows to arrange the first and second centering units 4, 40 to be arranged adjacent each other such that the protrusion 39 of the first centering unit 4 is insertable into the funnel shape 47 of the second centering unit 40.

The control unit 6 is adapted to operate the translational actuators 5, 50, 51, the gripping units 3, 30, the centering units 4, 40 in a fully automated manner for straightening and nesting two medical instruments (see Figs. 10A - 11H).

The figures 10A - 10H show a perspective view of a method using a loading unit 101 for centering and straightening of a first medical instrument 1, formed by a guidewire, by advancement and insertion into a second medical instrument 2, formed by a catheter. The figures 10A - 10H show a "backloading" method using the loading unit 101 in which a clinician is arranged proximal direction with respect to the first medical instrument 1 (see Fig. 10A - 10H, left side) and the patient is arranged in a distal direction with respect to the second medical instrument 2 (see Fig. 10A - 10H, right side).

The figure 10A shows the loading unit 101 which has a gripping unit 3 with two cylindrical rollers 33, 34 in a release configuration 31 in which two cylindrical rollers 33, 34 are spaced apart from a predefined axis P. The figure 10A show a first centering unit 4 in the open configuration 44 which has a distal conical protrusion 49. A second centering unit 40 in an open configuration 44 which has centering elements 41, 42 that are spaced apart along a predefined axis P is arranged distally from the first centering unit 4. A control unit 6 is adapted for converting the gripping unit 3 and the first and second centering units 4, 40 individually from each other between configurations by actuating translational actuators. The translational actuators are adapted to move the cylindrical rollers 33, 34 and halves of the centering units 4, 40 orthogonally to the predefined axis P toward and apart from each other for converting them. The second centering unit 40 is movable with respect to the first centering unit 4 by translational movement by a translational motion unit 50 for moving the second centering unit 40 along two struts 52, 53 in a direction parallel to the predefined axis.

The figures 10B and 10C show that the first gripping unit 3 is converted from the release configuration 31 to the gripping configuration 32 to engage a first medical instrument 1. The figures 10B and 10C further show that the first medical instrument 1 which has a bent distal tip was advanced across the first centering unit 4 in the open configuration 42. This is achieved by a translational actuation unit 5 formed by rotational actuators connected to the cylindrical rollers 33, 34 for translationally moving the medical instrument 1 by rotating the cylindrical rollers 33, 34 in opposing directions around their symmetry axis.

The figure 10D shows that the first centering unit 4 was converted from the open configuration 44 to the centering configuration 43. In the centering configuration 43 the two halves of the centering unit 4 engage each other to enclose the medical instrument 1 such that an enclosed section of the medical instrument 1 is aligned along the predefined axis P.

The figure 10E shows that the first medical instrument 1 is retracted by rotating the cylindrical rollers 33, 34 until the distal tip of the first medical instrument 1 is arranged completely enclosed within a distal conical protrusion 49 of the centering unit 4. Alternatively, to translationally moving the first medical instrument 1 relative to the first centering unit 4 the centering unit 4 may be moved with respect to the first medical instrument 1 and the gripping unit 3 in a distal direction for straightening the first medical instrument 1.

The loading unit 101 shown in Fig. 10E has a position sensor formed by a light barrier, a camera, or a capacitive sensor to verify via the control unit 6 if the first medical instrument 1 was retracted sufficiently far enough, such that the distal tip is arranged within the first centering unit 4 (not shown in the figures).

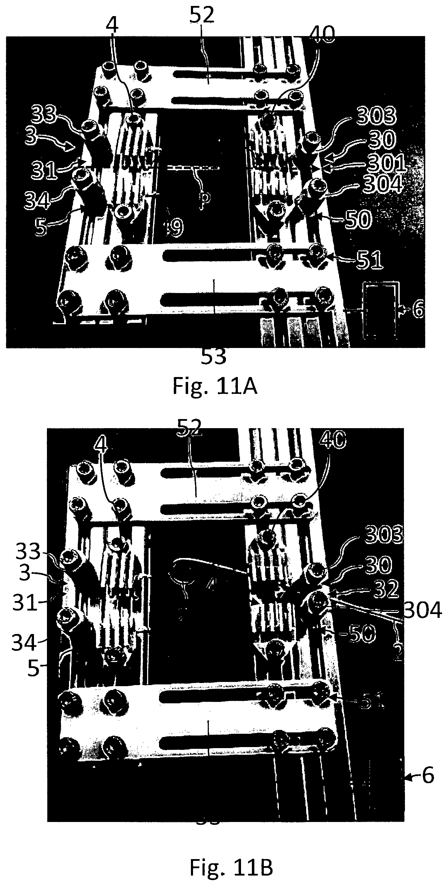

The figure 10F shows a second medical instrument 2 which is centered within the second centering unit 40 along the predefined axis P. A proximal end 21 of the second medical instrument 2 protrudes toward the distal conical protrusion 49 of the first medical instrument 1. In a preferred embodiment, the second medical instrument 2 may be positioned in the second centering unit 40 as described in Figs. 11A - 11D. A loading system comprising the loading unit of Figs. 10A-10H may further comprise a second centering unit 40 and also a second gripping unit 30 which is adapted for gripping and translationally moving the second medical instrument, e.g. via rotation of cylindrical rollers 303, 304 (see Figs. 11A - 11H).

The figure 10G shows that the second centering unit 40 is movably connected to the first centering unit 4 by a second translational motion unit 51 comprising a linear actuator for moving the struts 51, 52 on which the second centering unit is arranged. The second centering unit 40 is moved relative to the first centering unit 4 such that the distal conical protrusion 49 of the first centering unit 4 is advanced into a funnel shape of the second centering unit 40. Subsequently, the first medical instrument 1 can be advanced into the second medical instrument 2 by rotational actuators of the first translational motion unit 5 for driving the cylindrical rollers 33, 34.

The figure 10H shows that once the medical instruments 1, 2 were nested within each other, the second centering unit 40 can be moved in a distal direction by the second translational motion unit 51. The first centering unit 4 can be converted to the open configuration 44 again. The gripping unit 3 and the translational motion unit 5 may be used for advancing/retraction of the first medical instrument 1 for aligning the medical instruments prior to a surgical intervention or when carrying out a subsequent surgical intervention.

The conversion of the configurations 31, 32; 43, 44 of the gripping unit 3 and the centering units 4, 40 in Figs. 10A - 10H is controlled in a fully automated manner by the control unit 6 without requiring the presence of a clinician.

The figures 11A - 11H show a perspective view of a method using a loading system 102 comprising a loading unit 101 as described in Fig. 9 for centering and straightening a first medical instrument 1 for advancement and insertion into a second medical instrument 2. The figures 11A - 11H show a "front-loading" method using the loading system 102 in which a clinician is arranged in a proximal direction with respect the second medical instrument 2 (see Fig. 11A - 11H, right side) and the patient is arranged in a distal direction with respect to the first medical instrument 1 (see Fig. 11A - 11H, left side).

The figures 11A - 11H show a first and second gripping units 3, 30 in between which a first and second centering unit 4, 40 are arranged. The first and second gripping units 3, 30 have a first and second cylindrical roller 33, 34; 303, 304 respectively which are movable orthogonally relative to a predefined axis P to be converted between a release configuration 31 and a gripping configuration 32.

The first and second gripping units 3, 30 are connected to a first and second translational motion unit 5, 50 respectively. The first and second translational motion unit 5, 50 have rotational actuators for rotating the cylindrical rollers 33, 34, 303, 304 in opposing directions respectively for translationally moving the medical instruments 1, 2 along their longitudinal axis.

The first and second centering units 4, 40 in Figs. 11A - 11H are convertible between an open configuration 44 and a centering configuration 43 by moving the halves of the centering units 4, 40 toward and apart each other orthogonally to the predefined axis. In the centering configuration 43, the halves partially overlap in a direction parallel to the predetermined axis. Opposing wedge-shaped open shapes 45 (see Figs. 4A and 4B) form a channel for receiving medical instruments 1, 2. The centering units 4, 40 are arranged such that the channel for receiving the medical instruments 1, 2 is alignable with the predetermined axis P in the centering configuration 43. The first centering unit 4 has two opposingly arranged halves described in Fig. 4A with a conical proximal protrusion 49 and the second centering unit 4 has to opposingly arranged halves which have a funnel shape 47, 48 described in Fig. 4B.

The first gripping unit 3 and first centering unit 4 are movable in the direction parallel to the predetermined axis P with respect to the second gripping unit 30 and the second centering unit 40 by a third translational motion unit 51 which is formed by a translational actuator.

The conversion of the gripping units 3, 30, the centering units 4, 40 and operation of the translational motion units 5, 50, and 51 is controlled by the control unit 6 in a fully automated manner.

The figure 11A shows that the gripping units 3, 30 are in the release configuration 31, 301 and the centering units 4, 40 are in the open configuration 44.

The figure 11B shows that the second gripping unit 30 is converted from the release configuration 31 to the gripping configuration 32 to grip a second medical instrument 2 formed by a catheter. After or during gripping the second medical instrument 2, a spatial gripping position may be aligned with the predefined axis P. Subsequently, the second medical instrument 2 is retracted, such that a bent distal tip 21 of the second medical instrument 2 is retracted across the second centering unit 40 in the open configuration 44.

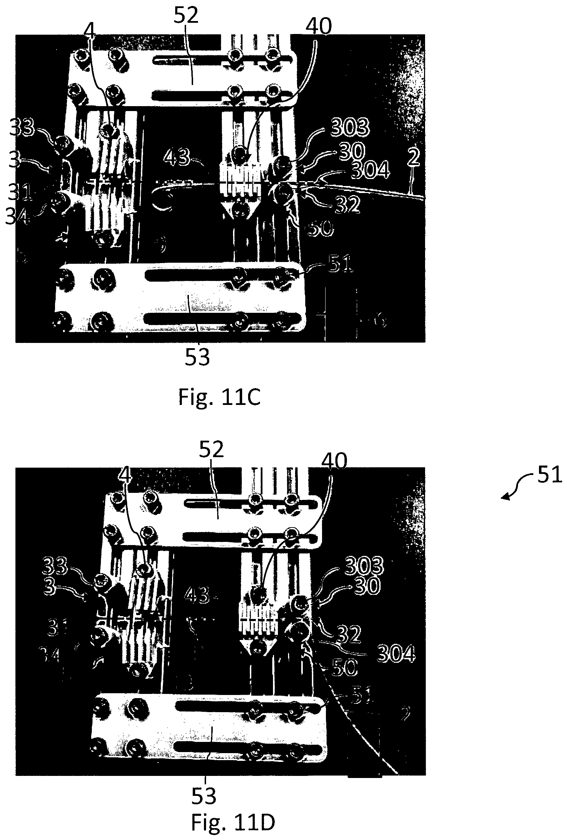

The figure 11C shows that the second centering unit 40 is converted to the centering configuration 43 to align a section of the second medical instrument 2 along the predefined axis P.

The figure 11D shows that the second medical instrument 2 which was retracted across the centering unit 40 as shown in Fig. 11C is advanced in a proximal direction by the opposing rotation of the cylindrical rollers 303, 304 until the distal tip 21 of the second medical instrument 2 is arranged within the second centering unit 40 and aligned along the predefined axis P. This allows the distal tip 21 to be straightened for nesting the medical instruments 1, 2.

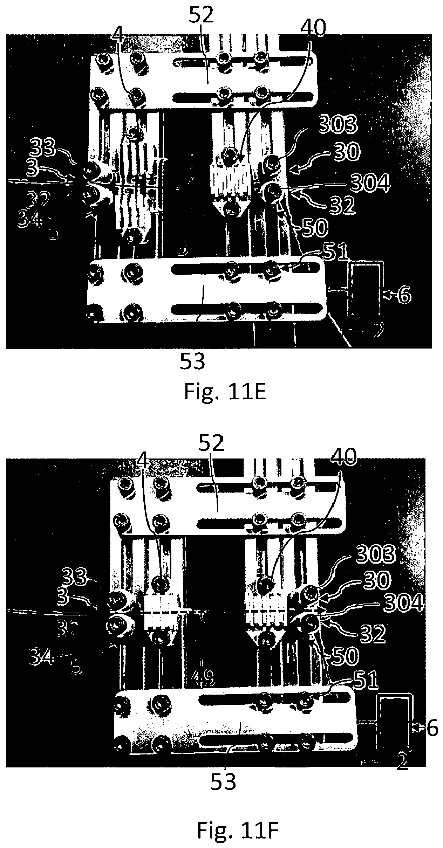

The figure 11E shows that the first gripping unit 3 is converted to the gripping configuration 32 to grip the first medical instrument 1 and then aligning its spatial gripping position with the predefined axis P. Subsequently, the cylindrical rollers 33, 34 of the first gripping unit 3 are rotated in opposing directions to translationally move the first medical instrument 1 formed by a guidewire along its longitudinal axis.

The figure 11F shows that the first centering unit 4 was converted to the centering configuration 43 and that the first medical instrument 1 was retracted. This allows that a proximal tip 11 of the first medical instrument 1 is arranged within the first centering unit 4 along the predefined axis P and straightening the proximal tip 11 of the medical instrument 1.

It would also be apparent to a person skilled in the art that the centering and straightening of the first and second medical instrument 1, 2 shown in Figs. 11A - 11E could be performed simultaneously operated by the control unit 6.

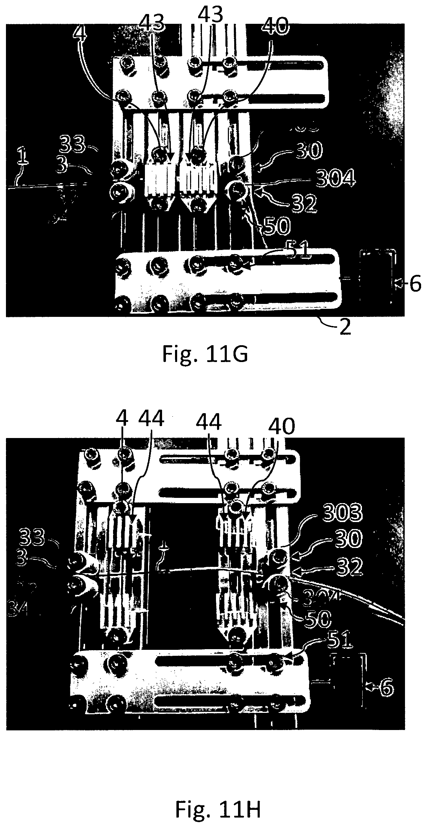

The figure 11G shows the insertion of the first medical instrument 1 in the second medical instrument 2 by moving the second gripping unit 30 and the second centering unit 40 with respect to the first gripping unit 3 and the first centering unit 4. The proximal conical protrusion 49 of the first centering unit 4 is inserted into the funnel shape (see Fig. 4B) such that the first medical instrument 1 can be easily advanced into the second medical instrument 2.

The figure 11H shows that after the insertion of the first medical instrument 1 into the second medical instrument 2, the centering units 4, 40 are moved apart from each other via the third translational motion unit 51 and converted to the open configuration 44 again to detach from the medical instrument 1, 2. The first and second gripping unit 3, 30 can still be used for moving the first and second medical instrument 1, 2 with respect to each other for further alignment or a subsequent procedure.

## Claims

1. A loading unit (101) for nesting medical instruments (1, 2) within each other, comprising
a gripping unit (3, 38) which is convertible between a gripping configuration (32) for radially gripping a first medical instrument (1) and a release configuration (31) for releasing the first medical instrument (1),
a translational motion unit (5) for translationally advancing and retracting the first medical instrument (1) in a substantially longitudinal direction (L) of the first medical instrument (1),
a centering unit (4) adapted for substantially aligning a longitudinal section of the first medical instrument (1), in particular together with the gripping unit (3, 38) and/or the translational motion unit (5), between the gripping unit (3, 38) and the centering unit (4) along a predefined axis (P),
a straightening means (8) for converting the first medical instrument (1), in particular a proximal or distal tip of the first medical instrument (1), from a curved configuration (13) to a straight configuration (14),
a control unit (6) for operating the straightening means (8), the translational motion unit (5), and the gripping unit (3, 38) for advancing a first medical instrument (1) in the straight configuration (14) into a cavity (9) of a second medical instrument (2) or over the second medical instrument (2).

2. The loading unit (101) according to claim 1, wherein the control unit (6) is adapted for controlling the translational motion unit (5) for successively advancing and retracting the first medical instrument (1) through the centering unit (4) and/or gripping unit (3, 38) for converting the first medical instrument (1) from the curved configuration (13) to the straight configuration (14).

3. The loading unit (101) according to one of the preceding claims, wherein the control unit (6) is adapted for converting the centering unit (4) between a centering configuration (43) for radially engaging and aligning the longitudinal section of the first medical instrument (1) in the centering unit (4) along the predefined axis (P) and an open configuration (44) for radially releasing the first medical instrument (1).

4. The loading unit (101) according to claim 3, wherein the centering unit (4) comprises a first and a second centering element (42) which are spaced apart from each other along the predefined axis (P), in particular arrangeable at different radial positions with respect to the first medical instrument (1), preferably on opposite sides of the first medical instrument (1), and adapted for engaging the first medical instrument (1) in the centering configuration (43).

5. The loading unit (101) according to any one of the preceding claims, wherein the translational motion unit (5) is adapted for driving the gripping unit (3, 38), in particular a pair of rotating rollers (33, 34) of the gripping unit (3, 38), for advancing and retracting the first medical instrument (1) in the longitudinal direction (L) and/or the translational motion unit (5) is adapted for moving the gripping unit (3, 38) with respect to the centering unit (4) and/or the centering unit (4) with respect to the gripping unit (3, 38).

6. The loading unit (101) according to any one of the preceding claims, wherein the loading unit (101) has a second translational motion unit (50) and/or second gripping unit (30) adapted for advancing and retracting
a core element (303) into a longitudinal lumen of the first medical instrument or
a sheath (304) which at least partially encloses the first medical instrument (1) circumferentially over the first medical instrument (1) for converting the first medical instrument (1) from the curved configuration (13) to the straight configuration (14).

7. The loading unit (101) according to any one of the preceding claims, wherein the control unit (6) is adapted for operating the straightening means (8) which interacts with a characteristic of the first medical instrument (1), in particular a magnetic characteristic, a temperature characteristic, an electric characteristic, or an electro-chemical characteristic, to transition the first medical instrument from the curved configuration (13) to the straight configuration (14).

8. The loading unit (101) according to any one of the preceding claims, wherein the gripping unit (3, 38) comprises or consists of at least one of
- a pinch gripper, in particular a pinch gripper formed by at least two cylindrical rollers, preferably having a centering surface, in particular a partially helical centering surface (35, 36), or two conical centering surfaces forming a V-shape, for radially engaging the first medical instrument (1) in the gripping configuration,
- a self-centering gripper (38) for engaging the first medical instrument with at least three contacting areas, in particular exactly three contact areas, of the self centering gripper (38) in the gripping configuration (32),
- a hook gripper which has an open shape in the release configuration (31), the open shape being configured for radially receiving the first medical instrument (1) in a predetermined position and an at least partially closed shape in the gripping configuration (32) for preventing radially releasing the first medical instrument (1), or
- a gripper for radially engaging the first medical instrument (1) at two different longitudinal locations of the first medical instrument (1) in the gripping configuration, in particular by a plurality of iris diaphragms, to center the first medical instrument (1), in particular along the predefined axis (P).

9. The loading unit (101) according to claim 8, wherein the self-centering gripper (38) has a mechanical coupling (38) for converting the self-centering gripper (385) by a rotational or translational motion between the gripping configuration (2) for gripping the first medical instrument (1) in which the first medical instrument (1) is contacted by the at least three contact areas (381, 382, 383) and the release configuration (31) for disengaging the first medical instrument (1).

10. A loading system (102) comprising a loading unit (101) according to one of the preceding claims,
a second gripping unit (30) which is convertible between a gripping configuration for radially gripping a second medical instrument (2) and a release configuration (301) for releasing the second medical instrument (2),
in a second translational motion unit for translationally advancing and retracting the second medical instrument (2) in a longitudinal direction (L2) of the second medical instrument (2), and
a second centering unit (40) adapted for aligning a longitudinal section of the second medical instrument (2), in particular together with the second gripping unit (30), between the second gripping unit (30) and the second centering unit (40) along the predefined axis (P),
a second straightening means (8) for converting the second medical instrument (2), in particular a proximal or distal tip of the second medical instrument (2), from a curved configuration (13) to a straight configuration (14),
the control unit (6) being adapted for operating the second straightening means (8), the second translational motion unit, and the second gripping unit (40) for nesting the first medical instrument (1) and the second medical instrument (2) within each other,
wherein the centering units (4, 40) are arranged or arrangeable between the gripping units (3, 30).

11. The loading system according to claim 10, wherein the centering units (4, 40), in particular together with the gripping units (3, 30), are movable along the predefined axis (P) with respect to each other, such that the straightened first medical instrument (1) arrangeable within the first centering unit (4) is insertable into the straightened second medical instrument (2) arrangeable within the second centering unit (40) by arranging the centering units (4, 40) adjacent each other.

12. Method for nesting medical instruments (1, 2) within each other using a loading unit (101), in particular a loading unit (101) according to one of the claims 1 to 9, comprising
a gripping unit (3) which is convertible between a gripping configuration (32) for radially gripping the first medical instrument (1) and a release configuration (31) for releasing the first medical instrument (1),
a translational motion unit (5) for translationally advancing and retracting the first medical instrument (1) in a longitudinal direction (L) of the first medical instrument (1),
a centering unit (4) adapted for aligning a longitudinal section of the first medical instrument (1), in particular together with the gripping unit (3), between the gripping unit (3) and the centering unit (4) along a predefined axis (P), wherein the method comprises the steps of:
- Converting the gripping unit (3) from the release configuration (31) to the gripping configuration (32) to radially grip the first medical instrument (1),
- translationally advancing the first medical instrument, in particular (1) via the translational motion unit (5), in the longitudinal direction of the first medical instrument (1) across the centering unit (4),
- optionally converting the centering unit (4) from an open configuration (44) to a centering configuration (43),
- translationally retracting the first medical instrument (1) via the translational motion unit (5) in the longitudinal direction of the first medical instrument (1), such that the first medical instrument (1), in particular a proximal or distal tip of the first medical instrument (1), is straightened by the centering unit (4),
- advancing the first medical instrument into a second medical instrument (2).

13. The Method according to claim 12, wherein the method comprises the following steps of:
- Converting a second gripping unit (30) from a release configuration to a gripping configuration (301) to radially grip the second medical instrument (2),
- Translationally retracting the second medical instrument (2) via a second translational motion unit in the longitudinal direction of the second medical instrument (2) across a second centering unit (40),
- optionally converting the second centering unit (40) from the open configuration (44) to the centering configuration (43),
- translationally advancing the second medical instrument (2) via the second translational motion unit in the longitudinal direction, such that the second medical instrument (2), in particular a proximal or distal tip of the second medical instrument (2), is straightened by the second centering unit (40),
- optionally moving the first and/or second centering unit, in particular together with the respective first and/or second gripping unit (3, 30), such that the centering units (4, 40) are arranged adjacent each other before the first medical instrument (1) is advanced into or onto the second medical instrument (2).
